(19) Europäisches Patentamt
European Patent Office
Office européen des brevets

(11) Publication number: 0 365 089 A2

(12) # EUROPEAN PATENT APPLICATION

(21) Application number: 89202593.3

(22) Date of filing: 13.10.89

(51) Int. Cl.[5]: C07D 333/18 , C07D 333/16 , C07D 333/24 , A61K 31/38

(30) Priority: 18.10.88 US 259373

(43) Date of publication of application: 25.04.90 Bulletin 90/17

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Biftu, Tesfaye
25 Victorian Drive
Old Bridge New Jersey 08857(US)

(74) Representative: Hesketh, Alan, Dr. et al
European Patent Department Merck & Co., Inc. Terlings Park Eastwick Road
Harlow Essex, CM20 2QR(GB)

(54) **2-Aryl-5(3-methoxy-5-(hydroxypropylsulfonyl)-4-propoxyphenyl) tetrahydrothiophen and analogs.**

(57) Analogs of 2-aryl-5-3-Methoxy-5-(2-hydroxypropylsulfonyl)-4-propoxyphenyl)tetrahydrothiophene were prepared.

These compounds are found to be leukotriene inhibitors and potent and specific PAF (Platelet Activating Factor) antagonists. They are therefore useful in the treatment of various diseases or disorders mediated by the leukotriene and/or PAF, for example, inflammation, cardiovascular disorder, asthma, lung edema, adult respiratory distress syndrome, pain, and aggregation of platelets.

EP 0 365 089 A2

# NEW 2-ARYL-5(3-METHOXY-5-(2-HYDROXYPROPYLSULFONYL)-4-PROPOXYPHENYL)-TETRAHYDROTHIOPHEN AND ANALOGS AS PAF-ANTAGONISTS AND LEUKOTIENE INHIBITORS

## BACKGROUND OF THE INVENTION

Platelet-activating factor (PAF) has recently been identified as an acetyl glyceryl ether phosphorylcholine (AGEPC), i.e., 1-O-hexadecyl/octadecyl-2-acetyl-sn-glyceryl-3-phosphorylcholine (Hanahan D.J., et al., J. Biol. Chem. 255:5514, 1980). Even before its chemical identification, PAF had been linked to various biological activities and pathways making it one of the important mediators responsible for a variety of physiological processes including activation or coagulation of platelets, pathogenesis of immune complex deposition, smooth muscle contraction, inflammation, pain, edema as well as respiratory, cardiovascular and intravascular alterations. Since these physiological processes are in turn associated with a large group of diseases, for example, inflammatory disease, cardiovascular disorder, asthma, lung edema, and adult respiratory distress syndrome, more and more scientific investigation has been focused on the search of a PAF-antagonist or inhibitor for treating or preventing these common diseases. Furthermore, the compounds of the present invention are found to be leukotriene inhibitors.

Substituted tetrahydrothiophenes can exist in six different stereoisomers as shown in Scheme I.

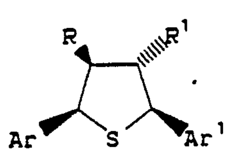

(1)

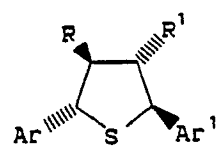

(2)

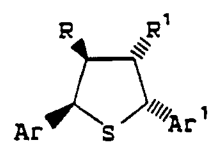

(3)

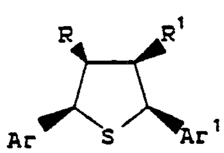

(4)

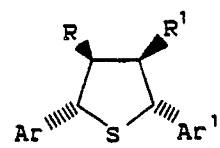

(5)

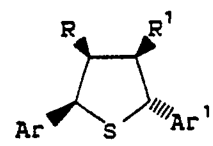

(6)

We have been able to prepare all the possible isomers of the tetrahydrothiophene analogs with different substituents and found that there exists a structure-activity relationship favoring the trans isomer of formula

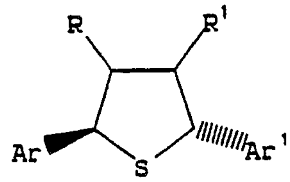

when R and $R^1$ are both hydrogen.

Accordingly, it is the object of the present invention to prepare the most potent isomers of novel tetrahydrothiophene derivatives as PAF-antagonists and leukotriene inhibitors and use them for the treatment of various diseases including prevention of platelet aggregation, hypertension, inflammation, asthma, lung edema, adult respiratory distress syndrome, cardiovascular disorder and other related skeletal-muscular disorders.

Another object of the present invention is to develop processes for the preparation of each and every stereoisomer of the 2,5-diaryltetrahydrothiophene analogs.

A further object of the present invention is to provide acceptable pharmaceutical compositions containing one or more of the tetrahydrothiophene derivatives and/or analogs as the active ingredient. As PAF antagonists and leukotriene inhibitors, these novel compositions should be effective in the treatment of various skeletal muscular related diseases.

Finally, it is the ultimate object of this invention to provide a method of treatment comprising the administration of a therapeutically sufficient amount of these PAF antagonist and leukotriene inhibitors to a patient suffering from various skeletal muscular disorders including inflammation, e.g., osteoarthritis, rheumatoid arthritis and gout, hypertension, asthma, pain, lung edema, or adult respiratory distress syndrome or cardiovascular disorder.

## DETAILED DESCRIPTION OF THE INVENTION

### A. Scope of the Invention

This invention relates to compounds of formula

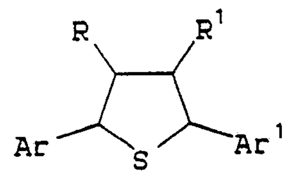

(I)

or a sulfoxide or sulfone thereof.
wherein R and $R^1$ independently are

(a) hydrogen;

(b) lower alkyl of 1-6 carbon atoms, e.g., methyl, cyclopropylmethyl, ethyl, isopropyl, butyl, pentyl or hexyl; or $C_{3-8}$ cycloalkyl for example, cyclopentyl;

(c) haloloweralkyl especially $C_{1-6}$ haloalkyl, for example, trifluoromethyl;

(d) halo especially fluoro;

(e) COOH;

(f) $CONR^2R^3$ wherein $R^2$ and $R^3$ independently represent $C_{1-6}$ alkyl and hydrogen;

(g) $COOR^2$;

(h) loweralkenyl especially $C_{2-6}$ alkenyl e.g., vinyl, allyl, $CH_3CH=CH\text{-}CH_2\text{-}CH_2$, or $CH_3(CH_2)_3CH=CH$-;

(i) $-COR^2$;

(j) $-CH_2OR2^2$;

(k) loweralkynyl especially $C_{2-6}$ alkynyl e.g., $-C{\equiv}CH$;

(l) $-CH_2NR^2R^3$;

(m) $-CH_2SR^2$;

(n) =O; or

(o) $-OR^2$;

(p) $-NR^2R^3$ Ar is

(a) phenyl or substituted phenyl of formula

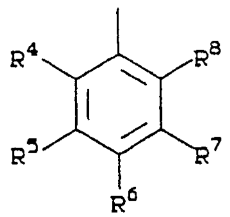

where $R^4$-$R^8$ independently represent H; $R^2$; YO- wherein Y is $R^2$, loweralkenyl such as $-CH_2-CH=CH_2$, loweralkynyl such as $-CH-C{\equiv}CH$, $-CH_2$-oxacyclopropyl, $-CH_2-C(O)OR^2$, $-CH_2-OR^2$, $-CH_2C(O)R^2$, $-CH_2CH(R)-R^2$, $-CH_2C_{3-8}$-cycloalkyl such as $-CH_2C_{3-6}$-cycloalkyl, $CH_2$-cyclopropyl, $-CH_2Ar$, and $-CH_2-CH(OH)-CH_2OH$; $R^2CH(R)CH_2SO_2$-; $R^2S$-; $R^2SO$-; $R^2SO_2$-; $CF_3O$-; $CF_3S$-; $CF_3SO$-; $ArCH_2SO_2$-; $CF_3SO_2$; $CH_3OCH_2-O$-; $R^2R^3N$-; $-OCH_2CO_2R^2$; $-SO_2NR^2R^3$; $-CO_2R^2$;$-CONR^2R^3$; $CONR^2$

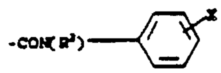

wherein X is halo,-C(O)Ar, $CF_3$, or $OR^2$; $-NR_2COR^3$; $-OCONH_2$; $-CR^2R^3R^9$; $-CH_2OR^2$; $-CH_2CO_2R^2$; $-CH_2OCOR^3$; $R^2CH(R)CH_2SO_7$-; $-NHCH_2COOR^2$; halo such as F, Cl, Br and I; $\overset{+}{N}R^2R^3R^9X^-$ wherein $X^-$ is an anion; wherein $R^9$ is the same as or different from $R^2$; $NR^2SO_2R^3$; $COR^2$; $NO_2$; or CN. For example, 3-methoxy-4-methylthiophenyl, 4-trifluoro-methoxyphenyl, 3-methoxy-4-trifluoro-methoxyphenyl, 3,4-dimethoxyphenyl, 3-methoxy-4-dimethylaminophenyl, 3,4,5-trimethoxyphenyl or $R^4$-$R^5$, $R^5$-$R^6$, $R^6$-$R^7$ and $R^7$-$R^8$ are joined together and form a bridge, for example, $-OCH_2O$-, $-OCH_2CH_2O$-,$-OCHR^2CHR^2S(O)_n$- wherein n is 0, 1 or 2 or $-OCH_2CH_2N$-; or

(b) monoheteroaryl, di- or polyheteroaryl, or fused heteroaryl containing from 1 to 3 of any one or more of the heteroatoms N, S or O in each heteroaryl ring thereof and each ring may either be unsubstituted or substituted appropriately with a radical selected from a group consisting of $R^4$-$R^8$, for example, pyridyl, pyrryl, thienyl, isothiazolyl, imidazolyl, tetrazolyl, pyrazinyl, pyrimidyl, quinolyl, isoqunolyl, benzothienyl, isobenzofuryl, pyrazolyl, indolyl, purinyl, carbozolyl, and isoxazolyl and the like;

(c) heteroarylalkyl;

(d) heterocycloalkyl; or

(e) heterocycloalkenyl; $Ar^1$ is

(a) 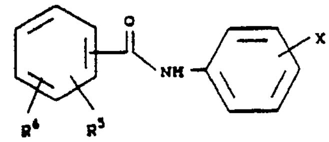 ;

(b) 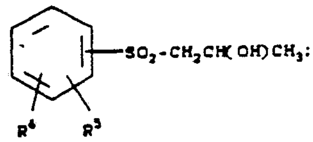 or

(c)

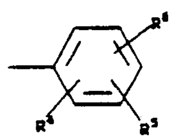

wherein $R^4$, $R^5$ and $R^6$ independently represents
YO- wherein Y is $-CH_2$-oxacyclopropyl, $-CH_2OR^2$, $-CH_2C(O)R^2$, $-CH_2CH(R)R^2$, $-CH_2Ar$ and $-CH_2CH(OH)-CH_2OH$; $R^2CH(R)CH_2SO_2-$;

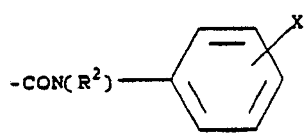

where X is halo, -C(O)Ar, $CF_3$, or $OR^2$; $-CH_2OR^2$; $-CH_2CO_2R^2$; $-CH_2COR^2$; $-NHCH_2COOR^2$; halo; $-N^+R^2R^3R^9X-$; or $R^4$-$R^5$ and $R^5$-$R^6$ joined together and form a bridge such as $-OCHR^2CHR^2-S(O)_n-$.

The following are the preferred heteroaryl groups:

(1) pyrryl or pyrryl substituted with $R^4$-$R^6$;
(2) furyl or furyl substituted with $R^4$-$R^6$;
(3) pyridyl or pyridyl substituted with $R^4$-$R^7$;
(4) thiophene or thiophene substituted with $R^4$-$R^6$;
(5) thiazolyl or thiazolyl substituted with $R^4$-$R^5$; or
(6) tetrazolyl or substituted tetrazols;
(7) pyrimidyl or pyrimidyl substituted with $R^4$-$R^6$;
(c) heteroarylalkyl such as 2-pyridylmethyl, 2-thienylmethyl and 3-isothiazolylethyl;
(d) heterocycloalkyl e.g., 1,3-dioxacyclohex-4-yl, piperidino, morpholino, oxacyclopropyl, pyrrolidino, tetrazolo, benzothiazolo, imidazolidino, pyrazolidino, and piperazino; or
(e) heterocycloalkenyl such as pyrrolino, 2-imidazolino, 3-pyrazolino or isoindolino.

Also, the sulfur of the tetrahydrothiophene could easily be converted to the corresponding sulfoxide or sulfone.

The compound of formula (I) can exist in the six isomers as described in Scheme I. These various isomers bear a close relationship to the PAF-antagonistic activity observed for the compounds within the scope of this invention.

Preferably, the PAF-antagonists of this invention are of structural formula

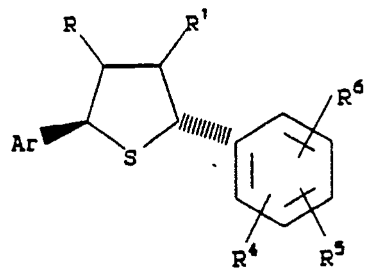

or an enantiomer or a salt thereof wherein R, $R^1$, $R^4$, $R^5$ and Ar are as previously defined, and $R^6$ is

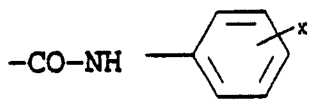

or $-SO_2CH_2CH(OH)CH_3$.

The most active PAF-antagonists discovered by us to date are the trans-2-(3,4,5-trimethoxyphenyl)-5-(3-methoxy-4-ethoxy-5-(3-chlorophenylaminocarbonyl)-phenyl)tetrahydrothiophene and trans-2-(3,4,5-trimethoxyphenyl)-5-(5-(2-hydroxypropylsulfonyl)-4-propoxy-3-methoxyphenyl)tetrahydrothiophene.

B. Preparation of the compounds within the scope of the invention

The PAF-antagonists of this invention have been prepared from diaroylbutanes as indicated in the following schemes.

The tetrahydrothiophenes were prepared from 1,4-diols or 1,4-dihalides by cyclization with appropriate reagents. The diols are in turn made by reduction of 1,4-diketones with the usual reducing agents. The diketones were made by oxidative coupling of enolates prepared from acetophenone or propiophenone derivatives, reaction of enolates generated from propiophenone or acetophenones with α-halo ketones or by the Stetter reaction as shown below in Scheme a.

## Scheme a

## Synthesis from diaroylbutanes, for example:

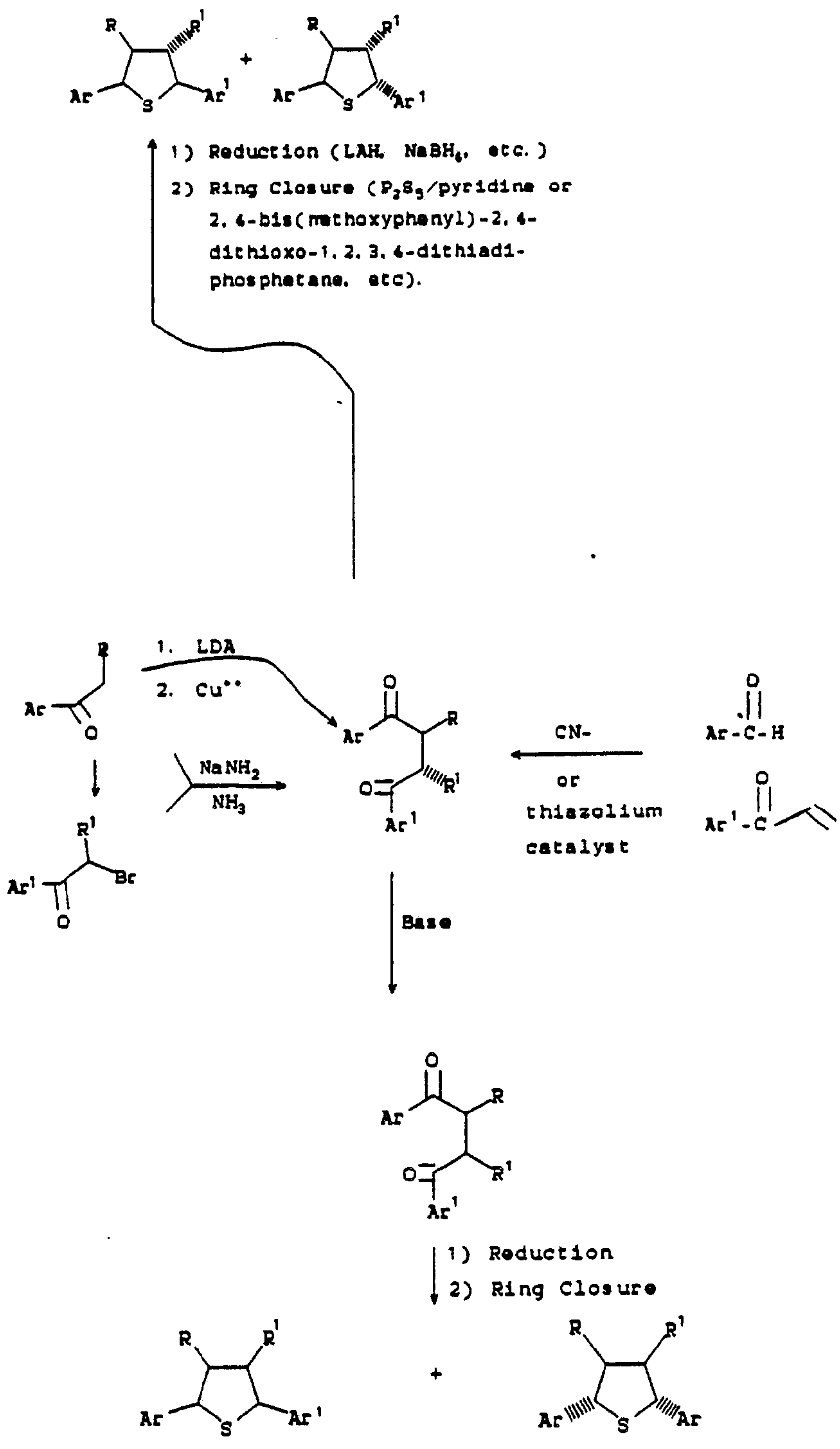


C. Utility of the compounds within the scope of the invention

This invention also relates to a method of treatment for patients (or mammalian animals raised in the dairy, meat, or fur industries or as pets) suffering from disorders or diseases which can be attributed to PAF

and/or leukotriene and more specifically, a method of treatment involving the administration of the compounds of formula (I) as the active constituents.

Accordingly, the compounds of Formula (I) can be used along other things to reduce pain and inflammation, to correct respiratory, cardiovascular, and intravascular alterations or disorders, and to regulate the activation or coagulation of platelets, the pathogenesis of immune complex deposition and smooth muscle contractions.

For the treatment of inflammation, cardiovascular disorder, asthma, or other diseases mediated by the PAF, the compounds of Formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc., the compounds of the invention are effective in the treatment of humans.

The pharmaceutical compositions containing the active ingredient may be in a form suitable for oral use, for example, as tablets, troches, lozenges. aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example

liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monoleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of Formula (I) are employed.

The amount of active ingredient that may be combined with the carrier materials to produce a single dosage form will vary depending upon the host treated and the particular mode of administration. For example, a formulation intended for the oral administration of humans may contain from 0.5 mg to 5 gm of active agent compounded with an appropriate and convenient amount of carrier material which may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

D. Bioassay Results Supporting the utility of the compounds of the present invention

It has been found that the compounds of formula (I) exhibit *in vitro* and *in vivo* antagonistic activities with respect to PAF:

A. *In Vitro* Assay:

*In vitro*, they inhibit PAF-induced functions in both the cellular and tissue levels by disturbing the PAF binding to its specific receptor site. The ability of a compound of formula (I) to inhibit the PAF binding to its specific receptor binding site on rabbit platelet plasma membranes was measured by an assay developed by us.

The inhibition of $H^3$-PAF binding to human or rabbit platelet plasma membrane by a PAF-antagonist of Formula (I) was determined by a method employing isotopic labeling and filtration techniques. Generally, a series of Tris-buffered solutions of the selected antagonist at predetermined concentrations were prepared. Each of these solutions contains 1 pmole of $^3$H-PAF, a known amount of the test antagonist, and a sufficient amount of the pH 7.0 Tris-buffer solution (10mM Tris, 0.25% bovine serum albumin, and 10 mM $MgCl_2$ per ml water) to make the final volume of 1 ml. After adding into a set of test tubes each with 100 $\mu$g of the platelet plasma remain suspension and one of the Tris-buffer solutions described above, the resulting mixture in each test tube was incubated at 0°C for about one hour or until the reaction was complete. Two control samples, one of which ($C_1$) contains all the ingredients described above except the antagonist and the other ($C_2$) contains $C_1$ plus a 1000-fold excess of unlabeled PAF, were also prepared and incubated simultaneously with the test samples. After the incubation was completed, the contents of each test tube were filtered under *vacuo* through a Whatman GF/C fiberglass filter and the residue washed rapidly several

times with a total of 20 ml cold (0°-5°C) Tris-buffer solution. Each washed residue was then suspended in 10 ml scintillation solution (Aquasol 2, New England Nuclear, Connecticut) and the radioactivity was counted in a Packard Tri-Carb 460CD Liquid Scintillation System. Defining the counts from a test sample as "Total binding with antagonist"; the counts from the control sample $C_1$, as "Total binding $C_1$"; and the counts from the control sample $C_2$ as "non-specific binding $C_2$", the percent inhibition of each test antagonist can be determined by the following equation:

$$\%\ \text{Inhibition} = \frac{(\text{Total binding } C_1) \text{ X} \begin{pmatrix}\text{Total binding} \\ \text{with antagonist}\end{pmatrix}}{\text{Specific binding}} \text{ X } 100$$

Specific binding =(Total binding $C_1$)-(non-specific binding $C_2$)

From our observation, compounds of formula (I) inhibit in vitro PAF-induced platelet aggregation (rabbit or human platelets); PAF-induced guinea pig peritoneal PMN (polymorphonuclear leukocytes) aggregation; PAF-induced human PMN secretion; and PAF-induced guinea pig smooth muscle contraction although these are not $H_2$-receptor antagonists. They are also shown in these inhibition studies to be highly specific to PAF. For example, they do not inhibit the binding of $H_1$ antagonist ($^3$H-pyrilamine) to guinea pig brain membrane, nor do they inhibit the binding of a cholecystokinin (CCK) receptor based on an assay on isolated rat pancreas membrane. Furthermore, they affect no or only minute inhibition on the histamine-induced ileum contraction from guinea pigs.

Results from the In Vitro assay

The antagonistic activity of the compounds of structural formula (I) is summarized in the following table:

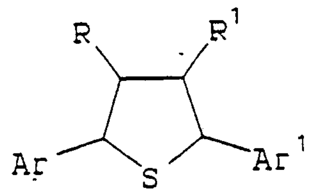

| R | R1 | Ar | Ar1 | dose(mM) | %inhibition |
|---|---|---|---|---|---|
| H | H | 3,4,5-trimethoxy phenyl | same as Ar | .03 | 69 |
| H | H | 3,4,5-trimethoxy phenyl | 5,6-di-methoxy-3-pyridyl | 5<br>1 | 96<br>80 |
| H | H | 3,4,5-trimethoxy phenyl | 3-(2-hydroxypropyl sulphonyl)-4-propoxy-5-methoxyphenyl | .03<br>.003 | 89<br>57 |
| H | H | 3,4,5-trimethoxy phenyl | (3-chlorophenyl-aminocarbonyl)phenyl | 300<br>30 | 47<br>6 |
| H | H | 3,4,5-trimethoxy phenyl | 3-carboxamido-4-propoxy-5-methoxyphenyl | 300 | 77 |
| H | H | 3,4,5-trimethoxy phenyl | 3-cyano-4-propoxy-5-methoxyphenyl | 30 | 27 |

B. In vivo Assay

Protocol for Assay of Oral Activity of PAF-antagonists in inhibiting PAF-induced symptoms including decreased arterial blood flow, increased vascular permeability and increased degranulation in rats

Animals:

Female, Wiston rats, 190-220 g

Procedure:

1.) Fast rats overnight.

2.) Weigh the rats the morning after fasting.

3.) Suspend a test compound in 0.5% methylcellulose with 12 ounce hand homogenizer or a sonicator if necessary to yield a fine suspension. Administer orally each of the rats with 2 ml of suspension such that the rat received a predetermined amount of the compound varying between 2 and 50 mg compound per kg.bodyweight of the rat.

4.) One hour after dosing, anesthetize the rat with sodium Nembutal (i.p.).

5.) One and a quarter hours after dosing, cannulate surgically the left femoral vein and artery of the rat.

6.) One and a half hours after dosing, infuse through cannulated vein 0.5 nannomoles (n moles) per 200 g body weight of the rat. Take blood samples from the cannulated femoral artery at 1.5, 3, 5, 8, 11, 15, 20 25 and 30 minute intervals. After the beginning of the PAF infusion as well as just before the PAF infusion, measure the following three parameters for each blood sample:

a) the arterial blood flow rate: determined by measuring the time to fill a pre-calibrated 14 µl capillary tube;

b) the vascular permeability: measured by calculating the increased hematocrit which results from loss of plasma from the circulation to extra-vascular spaces.

c) the circulatory degranulation: determined by assaying the increased plasma level of N-acetylglucosaminidase, a marker lysosomal enzyme.

7.) Determine the percent change in each parameter of a blood sample at each post-PAF interval including the 30 minute interval, relative to the pre-PAF blood values.

8.) Calculate the percent inhibition by the formula: % inhibition =

$$100\% \times \frac{\%\ \text{change without test compound} - \%\ \text{change with test compound}}{\%\ \text{change without test compound}}$$

Results:

Listed in the following table are the % inhibition of the PAF-induced responses at different oral doses of certain representative compounds.

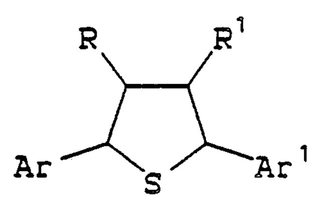

| R | $R^1$ | Ar | $Ar^1$ | Isomer | dose(mg/kg) | %inhibition A | %inhibition B |
|---|---|---|---|---|---|---|---|
| H | H | 3,4,5-trimethoxyphenyl | 3-(2-hydroxypropylsulfonyl)-4-propoxy-5-methoxyphenyl | | 1* | 60 | 62 |

A = increased vascular permeability

B = increased degranulation

* male rats weighing about 190-220g

The following examples illustrate the process for making the compounds of the present invention.

## EXAMPLE 1

Trans-2-(3,4,5-trimethoxyphenyl)-5-(3-methoxy-5-(2-hydroxypropylsulfonyl)-4-propoxyphenyl)-tetrahydrothiophene

Step A: Preparation of 1-(5-iodo-3-methoxy-4-propoxyphenyl)-4-(3,4,5-trimethoxyphenyl)-1,4-butanedione

A solution of 5-iodo-3-methoxy-4-propoxybenzaldehyde (26 g, 0.08 mol), 3,4,5-trimethoxyphenyl-2-(N,N-dimethylamino)ethyl ketone (18 g, 0.07 mol), and sodium cyanide (2.6 g, 0.053 mol) in DMF (250 mL) was stirred at 35 °C for 1.5 hr or until the reaction is complete as indicated by TLC. The reaction mixture was poured into ice cold 10% hydrochloric acid (2 L), and the precipitate was filtered, dried by suction, and taken up in dichloromethane (Note: if the solid is gummy and cannot be filtered easily it should be extracted with dichloromethane before filtration). The organic layer was washed with aqueous sodium bicarbonate and water, dried over anhydrous sodium sulfate, and filtered. The filtrate was evaporated to dryness and the residue crystallized from ether-hexane to provide 1-(5-iodo-3-methoxy-4-propoxyphenyl)-4-(3,4,5-trimethoxyphenyl)-1,4-butanedione in variable yields (25-60%; 24 g, [61%] was the maximum yield obtained); $R_f$ 0.4 ($SiO_2$, 2:1 [v/v] hexane-ethyl acetate); mp 111-112 °C.

Step B: Preparation of 1-(3-methoxy-5-methylthio-4-propoxyphenyl)-4-(3,4,5-trimethoxyphenyl)-1,4-butanedione

A mixture of metallic copper (10 g, 0.16 mol) and methyl disulfide (10 mL, 0.11 mol) in 2,4-lutidine (100 mL) was heated with stirring under nitrogen at 125 °C for 2 hr. 1-(5-iodo-3-methoxy-4-propoxyphenyl)-4-(3,4,5-trimethoxyphenyl)-1,4-butanedione (14 g, 0.03 mol) was added to the mixture, and the contents were heated at 160 °C for 16 hr. (Although the reaction appeared by TLC to be complete after 5-7 hr, the reaction was continued for 16 hr to ensure completion since the similar $R_f$ values of starting material and product precluded certainty as to completion.) The brown reaction mixture was filtered, and the filtrate was concentrated in vacuo at 55 °C. Dichloromethane (100 mL) was added to the residue, and the organic phase was separated and filtered through silica gel (100 g). The remaining solid was washed with hexane-ethyl acetate-dichloromethane (3:1:1 [v/v/v]). The combined filtrates were evaporated to dryness and the residue was treated with ethyl ether (500 mL) to provide crystals of 1-(3-methoxy-5-methylthio-4-propoxyphenyl)-4-(3,4,5-trimethoxyphenyl) 1,4-butanedione (8.85 g, 73%), mp 113-114 °C.

Step C: 1-(3-methoxy-5-methylsulfonyl-4-propoxyphenyl)-4-(3,4,5-trimethoxyphenyl)-1,4-butanedione

3-Chloroperbenzoic, acid (23 g, 0.13 mol) was added to a stirred solution of 1-(3-methoxy-5-methylthio-4-propoxyphenyl)-4-(3,4,5-trimethoxyphenyl)-1,4-butanedion (26 g, 0.056 mol) in dichloromethane (250 mL). After 2 hr at room temperature, the mixture was cooled and filtered to remove crystalline 3-chlorobenzoic acid, and the filtrate was evaporated to a small volume. Ethyl ether was added and the crystals were filtered and washed with 1:1 [v/v] ethyl ether-hexane to give the sulfone (24 g, 86%), mp 138-140 °C. An analytical sample was recrystallized from methanol, mp 145-146 °C.

Step D: 1-(3-methoxy-5-methylsulfonyl-4-propoxyphenyl)-4-(3,4,5-trimethoxyphenyl)-1,4-butanediol

Sodium borohydride (2.3 g, 0.06 mol) was added to a suspension of 1-(3-methoxy-5-methylsulfonyl-4-propoxyphenyl)-4-(3,4,5-trimethoxyphenyl)- 1,4-butanedione (23 g, 0.046 mol) in ethanol (300 mL), and the mixture was heated with stirring at 70 °C for 1 hr. The solution was cooled and dichloromethane (500 mL) and water (500 mL) were added sequentially. Hydrochloric acid (2.5 N) was added carefully until bubbling ceased. The organic layer was separated, washed with water, dried over anhydrous sodium sulfate, and evaporated to a syrup containing crude 1-(3-methoxy-5-methylsulfonyl-4-propoxyphenyl)-4-(3,4,5-trimethoxyphenyl)-1,4-butanediol. (23.5 g, quantitative yield), which was used directly in the next experiment without further purification.

Step E: Preparation of trans-2-(3,4,5 trimethoxyphenyl)-5-(3-methoxy-5-methylsulfonyl-4-propoxyphenyl)-tetrahydrothiophene

A solution of 2.7 g $P_2S_5$ suspended in 40 ml of pyridine was heated at 120° C for 1 1/4 hrs and then treated with 3.0 g of 1-(3-methoxy-5-methylsulfonyl-4-propoxyphenyl)-4-(3,4,5-trimethoxyphenyl)-1,4-butanediol and temperature reduced to 90° C and stirring continues for 1 1/2 hrs. After cooling and usual ether workup, the resulting oil (1.47 g) was chromatographed on HPLC using Partisil M20 column (hexane-ethyl acetate 60-40) to yield 130 mg of the cis isomer (m.p. 129-130°) and 280 mg of trans-2-(3,4,5-timethoxyphenyl)-5-(3-methoxy-5-methylsulfonyl-4-propoxyphenyl)tetrahydrothiophene as colorless oil.
NMR ($CDCl_3$): δ 1.05 (t, J=7.5 Hz, $CH_2CH_2CH_3$), 1.89 (m, $CH_2CH_2CH_3$), 1.89-2.49 (m, H-3, H-4), 3.26 (s, $SO_2CH_3$), 3.85, 3.89, 3.93 (3s, 4 $OCH_3$), 4.12 ($CH_2CH_2CH_3$), 4.81 (H-2,H-5), 6.68(s, Ar-H), 7.27 and 7.53 (Ar-H).

Step F: Preparation of trans-2-(3,4,5-trimethoxyphenyl)-5-(3-methoxy-5-(2-hydroxypropyl sulfonyl)-4-propoxyphenyl)tetrahydrothiophene

A solution of 200 mg trans-2-(3,4,5-trimethoxyphenyl)-5-(3-methoxy-5-methylsulfonyl-4-propoxyphenyl)-tetrahydrothiophene, in 2 ml THF treated with 0.35 ml 1.6 M nBuLi at -78° C and stirred for 15 min. and then treated with 40 ml of acetaldehyde and stirring continued for additional 30 min. The reaction mixture was quenched with two drops of water, evaporated and the residue was purified on a silica plate (ethyl acetate hexane 40-60) to give 182 mg of trans-2-(3,4,5-trimethoxyphenyl)-5-(3-methoxy-5-(2-hydroxypropylsulfonyl-4-propoxyphenyl)tetrahydrothiophene.
NMR ($CDCl_3$) δ: 1.22 (d, J=6Hz, $CH_3$), 1.44 (t, J=7 H3, $CH_3$), 1.8-2.6 (m, 4H), 2.2-2.7 (m, $SO_2CH_2$), 3.86, 3.89, 3.94 (3s, 4 $OCH_3$), 4.04-4.3 (m, $OCH_2$ and CH-O), 4.82 (m, H-2 and H-5, 6.68 (s, Ar-H), 7.2-7.54 (m, Ar-H).

## EXAMPLE 2

Trans-2-(3,4,5-trimethoxyphenyl)-5-(5-carboxamide-3-methoxy-4-propoxyphenyl)tetrahydrothiophene

Step A: Preparation of 1-(5-cyano-3-methoxy-4-propoxyphenyl)-4-(3,4,5-trimethoxyphenyl)-1,4-butanedione

A mixture of 1-(5-iodo-3-methoxy-4-propoxyphenyl)-4-(3,4,5-trimethoxyphenyl)-1,4-butanedione (20.0 g, 36 mmole) and CuCN (30.0 g, 330 mmole) was suspended in 200 ml dry N-methylpyrolidinone, heated to 140° C under nitrogen atmosphere for two hours. The dark reddish brown solution was cooled and poured into 500 ml of ice/water and extracted twice with 500 ml portions of $CH_2Cl_2$. The combined extracts were filtered through celite and then washed three times with 100 ml portions of saturated NaCl solutions. The methylene chloride solution was then filtered through a plug of silica (200 g) to remove red color. The filtrate was concentrated to a light orange color oil. It was crystallized from ethyl acetate/hexane to yield 13.5 g of 1-(5-cyano-3-methoxy-4-propoxyphenyl)-4-(3,4,5-trimethoxyphenyl)-1,4-butanedione.

Step B: Preparation of 1-(5-cyano-3-methoxy-4-propoxyphenyl)-4-(3,4,5-trimethoxyphenyl)-1,4-butanedione

A mixture of 1-(5-cyano-3-methoxy-4-propoxyphenyl)-4-(3,4,5-trimethoxyphenyl)-1,4-butanedione (13.5 g, 30 mmole) and $NaBH_4$ (2.0 g, 53 mmole) in 100 ml of absolute ethanol ard 25 ml ethyl acetate was stirred under nitrogen atmosphere. The temperature was raised to 40° C and maintained for one hour. The cooled reaction mixture was added to 200 ml icy water and was extracted twice with 300 ml portions of methylene chloride. The combined methylene chloride extracts were dried with anhydrous sodium sulfate and concentrated to a yellow oil (12.0 g, 89% yield). The product was used directly in the next reaction without further purification.

Step C: Preparation of trans-2-(3,4,5-trimethoxyphenyl)-5-(5-cyano-3-methoxy-4-propoxyphenyl)-tetrahydrothiophene

7.0 g of diol (obtained in the previous page), was dissolved in 100 ml pyridine and treated with 7.0 g Lawesson reagent and heated at 90° for 16 hours and solvent distilled off. The residue was then treated with 3N HCl (200 ml) and methylene chloride (200 ml). The organic layer was washed with water, 10% NaOH, water, dried over sodium sulfate (anhydrous) and evaporated to give 2.9 g of gummy residue. The residue was purified by chromatography over silica (ethyl acetate/hexane 40:60) to yield 920 mg of desired product.
NMR δ ($CDCl_3$): δ 1.06 (t, J=7Hz, $CH_2CH_2CH_3$). 1.82 (m, $CH_2CH_2CH_3$), 1.98-268 (m, H-3, H-4), 3.85, 3.90, 3.29 (3s, 4 $OCH_3$), 4.12 (t, $CH_2CH_2CH_3$), 4.78 (m, H-2, H-5), 6.70 (s, Ar-H), 7.22 (s, Ar-H)

Step D: Preparation of trans-2-(3,4,5-trimethoxyphenyl)-5-(5-carboxamide-3-methoxy-4-propoxyphenyl)-tetrahydrothiophene

60 mg of the above tetrahydrothiophene, was refluxed with 10 ml ethanol and 10 ml 10% NaOH for 4 hours and after the usual workup purified by prep TLC (Ethyl acetate-hexane 40-60, Rf 0.31). Yield 32 mg colorless gum.

## Claims

1. A compound of formula:

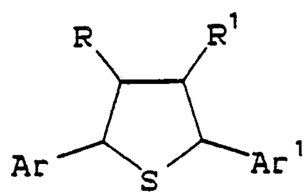

or a sulfoxide or sulfone thereof wherein R and R′ independently are
(a) hydrogen;
(b) $C_{1-6}$ alkyl or $C_{3-8}$cycloalkyl;
(c) halo $C_{1-6}$alkyl;
(d) halo;
(e) COOH;
(f) $CONR^2R^3$ wherein $R^2$ and $R^3$ independently represent $C_{1-6}$ alkyl and hydrogen;
(g) $COOR^2$;
(h) $C_{2-6}$alkenyl;
(i) $-COR^2$;
(j) $CH_2OR^2$;
(k) $C_{2-6}$alkynyl;
(l)$-CH_2NR^2R^3$;
(m) $-CH_2SR^2$;
(n) =O;
(o) $-OR^2$;
(p) $NR^2R^3$;
Ar is
(a) phenyl or substituted phenyl of formula

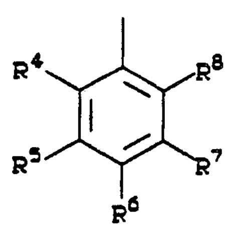

where $R^4$-$R^8$ independently represent H; $R^2$; YO- wherein Y is selected from a group consisting of $R^2$, $-CH_2$-oxocyclopropyl, $-CH_2C(O)OR^2$, $-CH_2OR^2$, $CH_2C(O)R^2$, $-CH_2CH(R)R^2$, $-CH_2C_{3-8}$cycloalkyl, $-CH_2Ar$ and $-CH_2$-$CH(OH)$-$CH_2OH$, $R^2CH(R)CH_2SO_2$-; $R^2S$-; $R^2SO$-; $CF_3O$-; $CF_3S$-; $CF_3SO$-; $CF_3SO_2$; $ArCH_2SO_2$-; $R^2R^3N$-; $-SO_2NR^2R^3$; $-CO_2R^2$;
$-CONR^2R^3$;

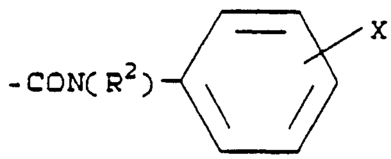

wherein x is halo, -C(O)Ar, $CF_3$, or $OR^2$; $-NR_2COR^3$; $-OCONH_2$; $CR^2R^3R^9$ wherein $R^9$ is the same as or different from $R^2$; $-CH_2OR^2$ $-CH_2CO_2R^2$; $-CH_2OCOR^3$; $R^2CH(R)CHR_2SO_2$-; $-NHCH_2COOR$; halo; or $N^+R^2R^3R^9X^-$ wherein $X^-$ is an anion; or $R^4$-$R^5$, $R^5$-$R^6$, $R^6$-$R^7$ and $R^7$-$R^8$ are joined together and form a bridge selected from a group consisting of $-OCH_2O$-, $-OCH_2CH_2O$-, $-OCHR^2CHR^2S(O)_n$- wherein n is 0, 1 or 2; and $-OCH_2CH_2N$-;
(b) monoheteroaryl, di- or polyheteroaryl or fused heteroaryl containing 1 to 3 of any one or more of the heteroatoms N, S (O)n or 0;
(c) heteroarylalkyl;
(d) heterocycloalkyl; or
(e) heterocycloalkenyl; and
$Ar^1$ is

(a) 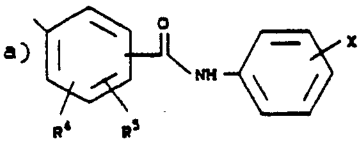 ;

(b) 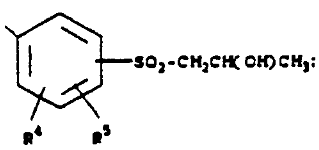 or

(c) 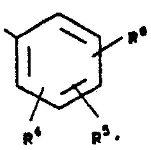

wherein $R^4$, $R^5$ and $R^6$ independently represent YO wherein Y is $-CH_2$-Oxacyclopropyl, $-CH_2OR^2$, $-CH_2C(O)R^2$, $-CH_2CH(R)R^2$, $CH_2Ar$ and $-CH_2CH(OH)CH_2OH$; $R^2CH(R)CH_2SO_2$-;

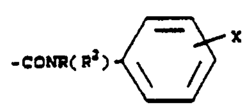

where X is halo. -(CO)Ar; $CF_3$, or $OR^2$ $-CH_2OR^2$; $-CH_2CO_2R^2$; $-CH_2COR^2$; $NHCH_2COOR^2$; halo; $-\overset{+}{N}R^2R^3R^9X$; or $R^4$-$R^5$ and $R^5$-$R^6$ joined together and form a bridge of $-OCHR^2CHR^2S(O)_n$-.

2. The compound of Claim 1 wherein the compound is of formula:

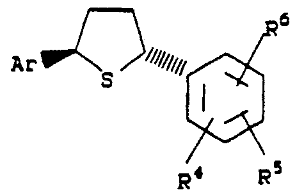

or an enantiomer or a salt thereof wherein R, $R^1$, $R^4$, $R^3$ and Ar are as previously defined; and $R^6$ is

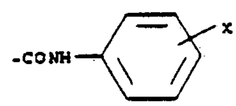

or $-SO_2CH_2CH(OH)CH_3$.

3. The compound of Claim 1 which is:

a) trans-2-(3,4,5-trimethoxyphenyl)-5-(3-methoxy-4-ethoxy-5-(3-chlorophenylaminocarbonyl)-tetrahydrothiophene; or

b) trans-2-(3,4,5-trimethoxyphenyl)-5-(5-(2-hydroxy)-propylsulfonyl-4-propoxy-3-methoxyphenyl)-tetrahydrothiophene.

4. A pharmaceutical composition for treating a disease or a disorder mediated by PAF comprising a pharmaceutical carrier and a therapeutically effective amount of a compound according to Claim 1.

5. The composition of Claim 4 wherein the active compound is:

a) trans-2-(3,4,5-trimethoxyphenyl)-5-(3-methoxy-4-ethoxy-5-(3-chlorophenylaminocarbonyl)-tetrahydrothiophene; or

b) trans-2-(3,4,5-trimethoxyphenyl)-5-(5-(2-hydroxypropylsulfonyl)-4-propoxy-3-methoxyphenyl)-tetrahydrothiophene.

6. The use of a compound as claimed in Claim 1 for the preparation of a medicament useful for the treatment of a disease or a disorder mediated by PAF.

7. The use as claimed in Claim 6 wherein the active compound is:

a) trans-2-(3,4,5-trimethoxyphenyl)-5-(3-methoxy-4-ethoxy-5-(3-chlorophenylaminocarbonyl)-tetrahydrothiophene; or

b) trans-2-(3,4,5-trimethoxyphenyl)-5-(5-(2-hydroxypropylsulfonyl)-4-propoxy-3-methoxyphenyl)-tetrahydrothiophene.

8. A process for preparing a compound of formula:

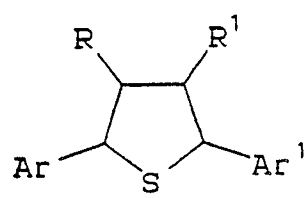

or a sulfoxide or sulfone thereof wherein R and R′ independently are

(a) hydrogen;
(b) $C_{1-6}$alkyl or $C_{3-8}$cycloalkyl;
(c) halo $C_{1-6}$alkyl;
(d) halo;
(e) COOH;
(f) $CONR^2R^3$ wherein $R^2$ and $R^3$ independently represent $C_{1-6}$ alkyl and hydrogen;
(g) $COOR^2$;
(h) $C_{2-6}$alkenyl;
(i) $-COR^2$;
(j) $-CH_2OR^2$;
(k) $C_{2-6}$alkynyl;
(l) $CH_2NR^2R^3$;

(m) $CH_2SR^2$;
(n) =O;
(o) $-OR^2$;
(p) $NR^2R^3$;
Ar is
(a) phenyl or substituted phenyl of formula

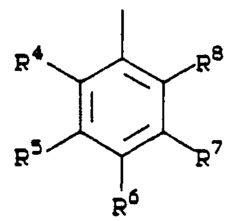

where $R^4$-$R^8$ independently represent H; $R^2$; YO- wherein Y is selected from a group consisting of $R^2$, $-CH_2$-oxocyclopropyl, $-CH_2C(O)OR^2$, $-CH_2OR^2$, $CH_2C(O)R^2$, $-CH_2CH(R)R^2$, $-CH_2C_{3-8}$cycloalkyl, $-CH_2Ar$ and $-CH_2$-CH(OH)-$CH_2OH$; $R^2CH(R)CH_2SO_2$; $R^2S$-; $R^2SO$-; $CF_3O$-; $CF_3S$-; $CF_3SO$-, $CF_3SO_2$; $ArCH_2SO_2$-; $R^2R^3N$-; $SO_2N^2R^3$; $-CO_2R^2$; $-CONR^2R^3$;

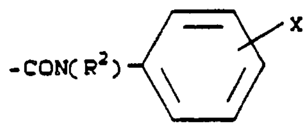

wherein x is halo, -C(O)Ar, $CF_3$, or $OR^2$; $-NR^2COR^3$ $-OCONH_2$, $-CR^2R^3R^9$ wherein $R^9$ is the same as or different from $R^2$; $-CH_2OR^2$ $-CH_2CO_2R^2$; $-CH_2OCOR^3$; $R^2CH(R)CH_2SO_2$-; $-NHCH_2COOR$; halo; or $N^+R^2R^3R^9X^-$ wherein $X^-$ is an anion; or $R^4$-$R^5$, $R^5$-$R^6$, $R^6$-$R^7$ and $R^7$-$R^8$ are joined together and form a bridge selected from a group consisting of $-OCH_2O$-, $-OCH_2CH_2O$-, $-OCHR^2CHR^2S(O)_n$- wherein n is 0, 1 or 2; and $-OCH_2CH_2N$-;
(b) monoheteroaryl, di- or polyheteroaryl or fused heteroaryl containing 1 to 3 of any one or more of the heteroatoms N, S (O)n or 0;
(c) heteroarylalkyl;
d) heterocycloalkyl; or
(e) heterocycloalkenyl; and
Ar is

(a) 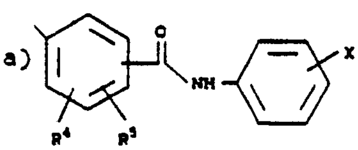 ;

(b) 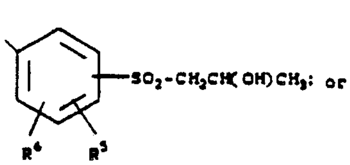 or

(c) 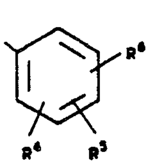

wherein $R^4$, $R^5$ and $R^6$ independently represent YO wherein Y is $-CH_2$-Oxacyclopropyl, $-CH_2OR^2$, $-CH_2C(O)R^2$, $-CH_2CH(R)R^2$, $CH_2Ar$ and $-CH_2CH(OH)CH_2OH$; $R^2CH(R)CH_2SO_2$-;

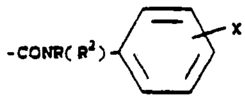

where X is halo. -(CO)Ar; $CF_3$, or $OR^2$ $-CH_2OR^2$; $-CH_2CO_2R^2$; $-CH_2COR^2$; $NHCH_2COOR^2$; halo; $-\overset{+}{N}R^2R^3R^9X$; or $R^4$-$R^5$ and $R^5$-$R^6$ joined together and form a bridge of $-OCHR^2CHR^2S(O)_n-$. comprising

(a) treating a compound of formula.

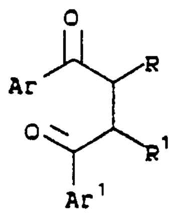

or

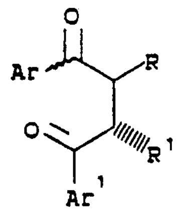

wherein Ar, $Ar^1$, R and $R^1$ are as previously defined.

with a reducing agent; and

(b) treating the product from Step (a) with $P_2S_5$ or 2,4-bis-(4-methoxyphenyl)-2,4-dithioxo-1,3,2,4-dithiadiphosphetane.

9. The process of Claim 8 wherein the compound to be prepared is a stereoisomer of formula:

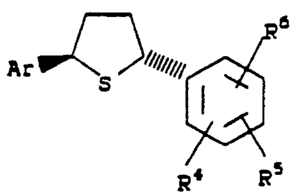

or an enantiomer or a salt thereof wherein R, $R^1$, $R^4$, $R^5$ and Ar are as previously defined; and $R^6$ is

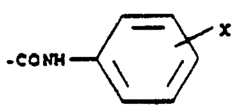

or $-SO_2CH_2CH(OH)CH_3$.

10. The process of Claim 9 wherein the compound to be prepared is:

a) trans-2-(3,4,5-trimethoxyphenyl)-5-(3-methoxy-4-ethoxy-5-(3-chlorophenylaminocarbonyl)-tetrahydrothiophene; or

b) trans-2-(3,4,5-trimethoxyphenyl)-5-(5-(2-hydroxy propylsulfonyl)-4-propoxy-3-methoxyphenyl)-tetrahydrothiophene.